# EUROPEAN PATENT SPECIFICATION

(11) **EP 2 317 968 B1**
(45) Date of publication and mention of the grant of the patent: **02.05.2018**
(21) Application number: 09777939.1
(22) Date of filing: 18.08.2009
(51) Int. Cl.: A61K 8/84, A61K 8/14, A61K 8/65, A61Q 19/10, A61K 8/73

(54) **LIPOSOMAL STRUCTURES FORMING ISOMETRIC AGGLOMERATES, ESPECIALLY BATH BEADS, THE PRODUCTION AND USE THEREOF**
ISOMETRISCHE AGGLOMERATE BILDENDE LIPOSOMALE STRUKTUREN, INSBESONDERE BADEPERLEN, IHRE HERSTELLUNG UND IHRE VERWENDUNG
STRUCTURES LIPOSOMALES FORMANT DES AGGLOMÉRATS ISOMÉTRIQUES, NOTAMMENT DES PERLES DE BAIN, LEUR PRODUCTION ET LEUR UTILISATION

(30) Priority: 01.09.2008 DE 102008045280
(43) Date of publication of application: 11.05.2011
(73) Proprietor: Merz Pharma GmbH & Co. KGaA, 60318 Frankfurt (DE)
(72) Inventor: EBINGER, Jürgen, 65510 Hünstetten (DE); BEUTLER, Rolf, 64739 Höchst -Hummetroth (DE); BECKER, Bärbel, 65719 Hofheim (DE)
(74) Representative: Müller, Claudia
(86) International application number: PCT/EP2009/005971
(87) International publication number: WO 2010/022873

(56) References cited:
- CA-A1- 1 077 851
- DE-A1- 19 757 059
- US-A- 4 183 959

## Description

### Field of the invention

The present invention relates to isometric agglomerates of a nearly spherically shaped form, especially bath beads, and the cosmetic, therapeutic and hygienic use thereof. The invention is especially concerned with agglomerates on the basis of carriers of natural origin such as collagen, gelatine, starch, cellulose or urea, which comprise both lipids and vesicle forming agents as well as emulsifying components and optionally additives, without impairing the stability or activity thereof.

The agglomerates when contacting water, especially in a bathing, will form liposomal mono- up to multi- layer structures thereby enabling an improved transport of active compounds. This will result in an improved balneological / or therapeutic action as well as a caring effect in the sense of a reconstruction of the skin protection barrier function.

### State of the art

Heretofore bath products have been mostly liquid preparations in the form of surfactant (tenside) solutions with the addition of ethereal oils and other active components, gels, especially for shower purposes, or in the form of dissolved salts. Among the liquid preparations, those tenside products which contain a proportion, - possibly a very high proportion- of oils and other fat-like products, are in a class by themselves. In a specific embodiment such bath oils may further contain vesicle forming lipids and water dispersible tensids (having an HLB = hydrophilic-lipophilic balance value of 6-13). Such bath oils are capable of the spontaneous formation of liposomes (see German Patent application No. DE 42 05 548 A1). However it is known, that pure O/W- (oil- in - water) tensides may impaire liposomal structures. Also known are solid bath additives, namely powders or granulates, comprising besides the CO₂- releasing compounds lipid compounds, W/O-(water-in-oil) and optionally O/W- surfactants (tensides) as well as vesicle forming components, and optionally suitable additives, cf. European Patent EP B 0930 064 B1. Such compositions address the problem to transfer the combinations of surfactants and lipids known from fluid bath preparations to solid bath preparations such as effervescent bath tablets although such compressed products, in order to maintain stability, may contain only small amounts of fluids and lipids. In fact the addition of liquid or pasty lipids and tensides adversely affects the flow and tableting and the galenically relevant properties of the compressed products with regard to friability, strength and solubility. However such flow and stability problems are particularly relevant with regard to the production of individually measured solid forms just as very small particulate spherical micropellets (isometrical agglomerates). By the term "isometric agglomerate", is usually intended a small, almost spherical solid form having an average particle size of about 0.5 up to 5 mm. Because of its condition it is difficult to achieve both a uniform application of substances as well as a continued stability while avoiding an undesired particle aggregation, particularly in case a specific delivering rate and - modus of desired compounds is intended. On the other hand there is a risk of hygroscopic changes in products containing salts. The US Patent US 4,093,745 discloses a non aggregating free- flowing bath bead composition by admixing urea prills with an oily emollient (emollients such as triglycerides or fatty acid alcohol esters). Optionally, in dependence of the desired purpose, powdered surfactants, especially sulphuric tensids, may be added. Additives such as colouring agents - which are especially desired because of the colouring of the bathing- may be applied in the form of a watery solution. In case additives are non -absorbable, they are first encapsulated into a water soluble powder such as starch or dextrin. However such material may lead to a sticky surface, which consequently may be covered by spraying coating surfactants onto that surface. However liposomal structures are not obtained.

Urea is a known substance which may also be used as moisturizing additive in smaller amounts, e.g. in water containing compositions, comprising specific lipids and active ingredients related thereto, optionally tensides as well as defined lipid transfer proteins, see DE 10 2005 056 538 A1.

However it were desirable if the aforementioned limitations and reductions in view of the stability or the flow properties of bath beads could be overcome and moreover liposomal structures within the bathing and with the application of water, respectively, were formed, in order to achieve an improvement of the skin caring as well as of optionally incorporated active ingredients, whereby in addition a highly comfortable management of the product were possible.

### Object of the invention

An object of the present invention therefore is to provide isometric agglomerates (such as spherical micropellets), in particular bath beads, especially on a natural basis, achieving an improvement concerning the active ingredient as well as skin caring effect without limitations concerning the stability and the application on the one hand and the activity on the other hand. Furthermore, when used these agglomerates shall produce liposomal structures spontaneously.

### Solution of the problem underlying the invention / Summery

These objects are achieved by the following subjects:
According to the invention there are provided isometric agglomerates especially having a nearly spherical form, in particular bath beads, the carrier of which preferably being composed of a material selected from the group comprising collagen hydrolyzate originating from plants, animals or marine products; gelatine or gelatine hydrolyzate, cellulose, starch or urea. Within the agglomerate such carrier comprises a mixture of one or more, particularly natural, liquid lipids and solid to waxy lipids as well as at least one vesicle forming component and an emulsifier mixture of at least one dispersable (dispersion) compound(s) and at least one O/W-compound(s) as well as optionally active ingredients and / or additives, selected from the group comprising conditioning agents, perfumes, dyes. Such agglomerates may be prepared in a mixing apparatus usually known for the production of tablets.

The invention insofar concerns isometric agglomerates having a nearly spherical form, and comprising:
80 to 97 %, or 80 to 96%, by weight of a carrier, selected from the group comprising collagen hydrolyzate originating from plants, animals or marine sources; gelatine or gelatine hydrolyzate; cellulose; carbohydrates, starch or urea; as well as
a) 0.1 % up to 10 % by weight (total amount of lipids) of
   a1) one or more liquid lipids;
   a2) one or more solid up to wax like lipids;
   or mixtures of a1) and a2);
b) 0.1 up to 2 % by weight of one or more vesicle forming lipids;
c) 0.1 up to 5, preferably up to 4% by weight of (of one or more of) a dispersion agent (dispersion component) having an HLB value of between 7 and 11;
d) 0.5 up to 6 % by weight of (one or more of) an O/W-emulsifier compound having an HLB value of especially between 13 and 40, more specifically between 13-25, and preferred of between 14 and 20.

It may also be preferred if the agglomerates comprise
e) 0 up to 10 %, preferably 0.1 up to 10 % by weight of additives selected from the group comprising perfumes, dyes, conditioning agents such as pearlescent agents, wax glossing agents, and / or
f) 0 up to 5 %, preferably 0.1 up to 5% by weight of active ingredients.
(all weight % in relation to the total amount of the composition if not otherwise indicated).

Surprisingly, when introduced into water, stable liposomal-, especially mono- up to multi layer structures (vesicles) are formed by the agglomerates and bath beads, respectively as described above which liposomal structures (vesicles) especially being useful in the transport of active ingredients or lipidic components. This could not be expected, the more so as there is a great amount of O/W- emulsifiers, which are considered as not being favourable in regard to the formation of liposomal structures. Furthermore, also solid lipids are applied. Moreover, it could not be expected, that liposomal structures may be formed within the bathing, since the lipid is adsorbed or even absorbed within the core of the pellet, and since specific requirements concerning the emulsifiers are necessary to deliberate the vesicle forming lipids differing from those requirements to deliberate the lipids. It was therefore also especially surprising since - starting from vesicle forming granulates or powders - there is presently a completely different surface condition in the present agglomerates with respectively different conditions concerning adsorption and absorption.

### Description of the figures

In the present figures 1 to 4 the situation is represented in trans electron photomicrographs (TEM photographs) taken in a bath after the addition of the product of Example 3 (Figures 1 and 2) and example 5 (Figures 3 and 4), in which the multi-layer- structures surprisingly formed are shown.

### Detailed description of the invention

The agglomerates according to the present invention in particular comprise at least 80 % by weight, preferably 80 to 97 % by weight, and more preferred 85 to 96 % by weight of the carrier material. The carrier material is selected from natural, especially water - soluble base materials such as collagen hydrolyzate, e.g having a molecular mass of at most 50 000 D; which can be obtained from plant, animal or marine sources; gelatine / gelatine hydrolyzate, having a molecular mass of about 1 500 up to 250 000 D, preferably 50 000 D up to 250000 D; cellulose having an average molecular mass of 50 000 D up to 500 000 D, preferably 50 000 D up to 250 000 D; carbohydrates such as saccharose, fructose, dextrose; starch (e.g. rice starch, wheat starch, corn and potato starch), or else urea. A preferred carrier material is urea. The water - soluble carriers themselves are essentially anhydrous (with probably a very low amount resulting from the production) and are of an essentially spherical form.

Within the agglomerates according to the invention such carriers comprise a lipid phase (lipid layer). The lipids are selected from the group comprising liquid lipids a1) (natural oils) as well as solid to semisolid (wax like) lipids a2) such as hydrogenated natural oils, or mixtures of a1) and a2). Preferred lipids are selected from mixtures of one or more lipids according to a1) and one or more lipids according to a2) (for example mixtures of one ore more liquid lipids a1) and one or more solid / wax like lipids a2)), for example in a ratio of 1:1 to 1:7. The liquid lipids (oils) are selected from the group comprising:
Olive oil, sunflower oil, soy oil, peach pit oil, apricot pit oil, grape seed oil, castor bean oil, peanut oil, almond oil, mink oil, wheat germ oil, sesame oil, thistle oil, almond oil, avocado oil, shea butter or illipé butter; natural liquid waxes, e.g. jojoba oil or its substitute, oleyl erucate; coconut oil, borage oil, corn oil, walnut oil, palm (germ) oil, macademia nut oil, hazelnut oil, rosehip oil, cotton seed oil, papaya (paw paw) oil, or mixtures thereof.

Preferred liquid lipids (plant oils) are selected from (the group comprising) soy oil sunflower oil, wheat germ oil, olive oil, or mixtures thereof.

The preferred solid to wax like lipids a2) are preferably selected amongst hydrogenated soy oil, hydrogenated palm oil, hydrogenated caster oil, hydrogenated sunflower oil.

Also hydrogenated macademia nut oil, hydrogenated coconut oil may be suitable. The lipids are preferably present in a total amount of from 0.1 up to 10% by weight, especially 0.5 up to 5 % by weight. In case of mixtures of both liquid and solid lipids, an excess of the solid lipids vis a vis the liquid lipids is preferred.

Preferred solid lipids are particularly selected from (the group comprising) hydrogenated soy (bean) oil, hydrogenated palm oil and hydrogenated sunflower oil, or mixtures thereof.

The water - soluble carriers within the agglomerates according to the invention further comprise an emulsifier combination comprising (at least one) a dispersion component (agent) c) and (at least one) an O/W- emulsifier component d). Thereby an excess of the O/W -emulsifier is especially preferred.

Preferably, the ratio between the O/W- emulsifier(s) d) and the dispersion component(s) (agent) c) is greater > than 1, especially 1:2 up to 1:8, in particular 1:4 up to 1:7.

The dispersion components (agents) c) are preferably chosen amongst those substances, having an HLB- value of between 7 and 11, preferably 7.5 to 10. Such compounds are in particular non ionic substances such as fatty alcohols such as cetearyl alcohol, or monoglycerides such as glycerol stearate; furthermore lower polyethoxylated or lower polypropoxylated and mixed lower polyoxylated C₈₋₂₂ fatty alcohols having an ethoxylation and / or propoxylation degree (EO- PO degree) of between 1 and 5 (EO /PO). Preferred compounds are low poly(eth)oxylated C₈₋₂₂ fatty alcohols having an ethoxylation degree (EO- degree) of between 1 and 5. Such compounds may be selected from macrogollaurylethers having 1 to 4 EO units such as laureth-2, laureth 3, laureth-4 (polyoxyethylene lauryl ether having 2, 3 and 4 EO units, respectively), or mixtures thereof.

Suitable O/W emulsifiers d) may particularly be selected from those having an HLB value of preferred 13 and 40, preferably 13-25, more preferred 13.5 to 20, and especially 13.5 to 17. These are in particular higher polyethoxylated (EO) and / or higher polypropoxylated (PO) or higher mixed (EO / PO) polyoxylated C₈₋₂₂ -fatty alcohols, (especially alkyl-C₁₀₋₁₈ fatty alcohol alkoxylates), or such ethoxylated and / or propoxylated C₁₂₋₂₂ fatty acids having ethoxylation or propoxylation degrees of 8-25, or mixtures thereof, such as (HLB value in parentheses): polyoxypropylene-(15)stearylether, or polyoxyethylenesorbitane-monooleate (15,0), polyoxyethylene-sorbitan monostearate or - palmitate (15,0), polyoxyethylene stearylether (15,3), polyoxyethylene oleylether (15,3), polyoxyethylene-oxypropylene monostearat (15,7), poyloxyethylene- fatty alcohol ether (16,0), polyoxyethylene monostearat (HLB 15) and the like. Especially preferred emulsifiers are ethoxylated C₁₂₋₂₂ fatty acids or ethoxylated C₈₋₂₂ fatty alcohols having ethoxylation units of between 10 to 20 (HLB value e.g. 13-20, preferably 13.5 to 18).

Further suitable emulsifiers are sugar esters and sugar ethers of C₁₂₋₂₂ -fatty acids or of C₈₋₂₂ -fatty alcohols. These are for example sugar esters of saturated, unsaturated, partially saturated C₁₂₋₁₈ -fatty acids; polyoxyethylated and / or polyoxypropylated sugar esters of C₁₂₋₁₈ -fatty acids, with an EO and PO degree, respectively of between 8 and 25. Sugar ethers may be selected from those ethers of saturated, unsaturated, partially saturated C₈₋₂₀ -(or C₈₋₂₂)-fatty alcohols; or of polyoxyethylated and / or polyoxypropylated sugar ethers of these C₈₋₂₀- fatty alcohols. Thereby, the EO- and PO- degree respectively may be between 8 and 25. The sugar part may preferably be selected from the group comprising glucose, methyl glucose, saccharose and the acids and alcohols may preferably be selected from ₁₆₋₁₈ -fatty acids / and - alcohols. Preferred compounds are ethoxylated compounds. The acid- and alcohol components respectively, are specifically derived from aliphatic carboxylic acids / - alcohols. These are in particular caprylic acid, caprine acid, lauric acid, myristic acid, palmic acid, stearic acid, oleic acid, and mixtures thereof, as well as such fatty alcohols, respectively (oleylalcohol, laurylalcohol, stearylalcohol).

In a further especially preferred embodiment the O/W emulsifier components d) are selected amongst anionic compounds, especially such as (alkyl) C₁₂₋₂₂ -fatty alcohol - amino acid ester compounds, preferably an alkali salt thereof, whereby the amino acids are selected from the group comprising glycine, taurine, such as oleyl-, lauryl-taurate, sodium methyl oleyl- taurate and the like. These may be used in the form of their salts, such as the alkali salts.

The O/W emulsifier components d) may preferably be present in an amount of between 0.5 up to 6% by weight, the dispersion component (agent) c) may preferably be present in an amount of between 0.1 % up to 5 % by weight. The total amount of the emulsifier mixture (c) + d)) is between 0.6 and 11%, especially 0.6 up to 6% by weight.

Especially preferred dispersion components (agents) and O/W- emulsifier components c) and d) are selected from the group comprising low-polyethoxylated or low polypropoxylated and mixed low polyoxylated (PO/EO) C₁₂₋₁₈ -fatty alcohols having an EO / PO degree (ethoxylation / propoxylation degree) of between 1 and 5, preferably 2 to 4; especially having an EO degree of between 2 to 4. particularly such as the said lauryl ether compounds; as well as from C₁₂₋₁₈ -fatty alcohol-taurates, especially sodium-methyl oleyl-taurate.

The agglomerates according to the present invention do further comprise vesicle forming substances b). These are selected from phospholipids such as lecithin (e.g. egg or soy lecithin), or phosphatidyl-choline, -serine, or -diethanolamine, phosphatidylinosite, e g. derived from soy, rape, cotton seed, egg; sphingolipids (e.g. ceramides, cerebrosides, sphingosine, sphingomyelin), or ethoxylated sterols such as phytosterols (mixtures of sistosterol, camposterol and stigmasterol), for example the ethoxylated derivatives thereof such as PEG-(polyethyleneglykol) -5- soy bean sterol or PEG- (polyethyleneglykol) -5 rapeseed sterol = General® 122 E5 and General® R E5, respectively.

The amount of the vesicle forming substance*(s)* is preferably between 0.1 and 5 % by weight, especially between 0.1 and 3 % by weight.

Especially preferred vesicle forming substances are phospholipids, and amongst these lecithins, in particular soy lecithin, egg lecithin, or mixtures thereof.

The agglomerates according to the invention can further (optionally) contain an amount of active ingredients f) and optionally an amount of additives e), selected from the group comprising perfumes, dyes, conditioning agents.

Suitable active ingredients, which - if present- may be present in an amount of between 0.1 up to e.g. 5 % by weight, especially include ethereal oils, plant extracts, or mixtures thereof. These active ingredients may representatively comprise ethereal oils / extracts suitable for cosmetic or optionally medical bath additives, preferably selected from kaki leaves, mango, figs, lavender, cedar, lotus flowers, chamomile flowers, ylang ylang, ginkgo, pine needles, cypress, orange, rose wood, extracts from plum, birch-leaf extract, aloe-vera extract, extracts from marigold, hibiscus, burdock, witch hazel-, march pennywort leaf, algae, quince, water lily and cinnamon, extracts from thyme, mint, limes, grapefruit, mandarin, juniper, valerian, lemon balm, eucalyptus, palma-rosa, rosemary, lemon grass, wild rose, spruce needles and pine needles, ginger, red-currant, lime-blossom, marigold, magnolia, pineapple, guava, Echinacea, ivy-leaf, blueberry, kaki (persimmon diospyros), melone- extract and the like or mixtures thereof.

These extracts can be produced, for example by steam distillation. Hereby there are obtained from the cited plants, for example, ethereal oils which are particularly preferred. The extracts can also be obtained by other known ways, for example by solvent extraction (with alcohols, triglycerides or hydrocarbons, water and mixtures thereof) and then be used as such.

Examples of analogous synthetically produced substances are terpenes and terpenoids such as camphor, menthol, cineol or mixtures thereof.

Other suitable active ingredients include for example, vitamins, such as Vitamin A, E, or others and suitable derivatives thereof, such as esters, e.g. palmitate, acetate, or phosphate. Also, essential unsaturated fatty acids and their esters may be used, such as linol- or linolen acid, glyceryl linoleate, glyceryl linolenate.

Furthermore, active substances that promote blood circulation, such as nicotinic acid derivatives such as methyl nicotinate or tocopheryl nicotinate, alpha- and beta-hydroxy acids and their derivatives, e.g. glycolic acid, malic acid, citric acid, tartaric acid, lactic acid, salicylic acid, isopropylbenzyl salicylate, C12-13 alkyl lactate (Cosmacol® ELI), or else antiphlogistic (anti-inflammatory) and antibacterial substances such as triterpenes, e.g. ursolic acid, glycyrrhizinic acid or glycyrrhetinic acid, and their derivatives, e.g. stearyl glycyrrhetinate, potassium glycyrrhinate; pantothenic acid derivatives, e.g. D-panthenol, panthenyl triacetate; or also polyphenols, flavonoids, e.g. rutine, ferulaic acid, and their esters, or isoflavones such as soybean isoflavones or co-enzyme Q 10, can be used as active substances. Preferred active ingredients are selected amongst ethereal oils, plant extracts, vitamins or mixtures thereof.

A further group of active ingredients includes skin -care agents. These may be selected amongst glycerine, propylene glycol or polyethylene glycols, polypropylene glycol, butylenes glycol, sorbitol or polymers such as of polyquaternium types, collagen, the hydrolyzates thereof, amino acids.

Besides these acitive ingredients e) or together with these also further additives d) as mentioned above may be incorporated into the agglomerates according to the invention, preferably in an amount of between 0.1 and 10 % by weight. These include conditioning agents, dyes and perfumes. The conditioning agents may be selected amongst mono- and dialkylphosphates or cetyl alcohol, or the like, or mixtures thereof. Especially suitable conditioning agents are colloids. These may be selected amongst polysaccharides (e.g. xanthan gum), dextrines, cyclodextrine or similar colloidal saccharides, furthermore high dispersible silica acid compounds such as silicium dioxide, or albumines, or mixtures thereof, or water - soluble (poly)(meth)acrylate polymers such as carbomers, or water- soluble cellulose derivatives such as methyl cellulose, hydroxypropyl cellulose or hydroxyethyl cellulose, hydroxypropyl methyl cellulose, whereby the colloids mentioned last, may have a molecular mass of more than 40 000 D, for example carbomers: 1,25 up to 4 millions D.

Another group of suitable conditioning agents includes pearlescent agents, glossing agents on a waxy base (wax glossing agents), e.g. beeswax, and solubilizing agents, respectively, such as ethanol, isopropanol, (poly)ethylene glycol, (poly)propylene glycol.

Dyes which can be used in compositions according to the invention may be selected amongst water - soluble compounds known in the art for that purpose. Suitable perfumes may be selected amongst ethereal oils such as mentioned above or other analogous / synthetic compounds known in the art.

The amounts of dyes and perfumes are within the range usually applied for such compounds and are known in the art.

The conditioning agents may be applied in amounts of e.g. from 0.1 up to 5% by weight.

Preferred additives are selected from the group comprising dyes, perfumes colloids, especially silicium acid- (silicium dioxide) compounds, pearlescent agents, glossing agents on a waxy basis (e.g. beeswax), or mixtures thereof.

### Preferred embodiments according to the invention

Especially preferred agglomerates are those wherein the carrier is selected from urea, especially spherically formed urea beads (prills).

Furthermore
- the liquid lipid(s) (oils) a1) is / are selected from the group comprising olive oil, sunflower oil, soy (bean) oil, peach pit oil, apricot pit oil, grape seed oil, castor oil, peanut oil, almond oil, mink oil, wheat germ oil, sesame oil, thistle oil, avocado oil, shea butter or illipé butter; natural liquid waxes, e.g. jojoba oil or its substitute, oleyl erucate; coconut oil, borage oil, corn oil, walnut oil, palm oil, macademia nut oil, palm germ oil, hazelnut oil, rosehip oil, cotton seed oil, papaya (paw paw) oil, or mixtures thereof.
- else preferably the solid lipid(s) a2) is /are selected from hydrogenated soy(bean)oil, hydrogenated palm oil, hydrogenated castor oil, hydrogenated sunflower oil or mixtures thereof.

A further advantage is a mixture of one or more lipids (e.g. above mentioned oils) a1) and one or more of the above mentioned lipids a2).

In another advantageous embodiment according to the invention the dispersion agent(s) c) is / are selected amongst non ionic low polyethoxylated or low polypropoxylated and low - mixed polyoxylated, preferably low ethoxylated C₈₋₂₂ - fatty- alcohols having an ethoxylation (and / or propoxylation degree) (EO/PO-degree) of between 1 and 5, ethoxylated glyceroles, being esteryfied with C₁₂₋₂₂ - fatty- acids and having an EO- degree of between 0.5 and 3.

In a further preferred embodiment, the O/W emulsifier(s) d) within the agglomerates as described is /are selected from the group comprising ethoxylated C₁₂₋₂₂ -fatty-acids or ethoxylated C₈₋₂₂ -fatty- alcohols having ethoxylation degrees of between 13 and 18 and an HLB- value of between 13 and 18, especially 14-18; sugar esters or sugar ethers of C₁₂₋₂₂ -fatty- acids or of C₈₋₂₂ -fatty- alcohols; polyoxyethylated sugar esters of C₁₂₋₁₈ -fatty- acids, having an EO- degree of between 8 and 25 and the sugar being derived from glucose, saccharose, methyl glucose; as well as alkyl C₁₂₋₂₂ -fatty- alcohol- amino acid ester- compounds, especially an alkali metal salt thereof, the amino acid being selected amongst glycine and taurine.

The ratio between the dispersion agent(s) c) / O/E-emulsifier*(s)* d) is preferably as indicated, in particular 1:2 to 1:8, and more specifically 1:3 to 1:7.

In another preferred embodiment according to the invention the amount of the solid to waxy like lipid(s) a2) is higher than die amount of the liquid lipid(s) a1), particularly 1,5:1 to 5:1.

In the invention the agglomerates comprise a vesicle forming lipid or lipids which is / are selected amongst phospholipids, such as lecithin (egg- or soy lecithin), phosphatidyl-cholin, -serin or -diethanolamine; phosphatidylinosit, e.g. derived from soy, rape, cotton seed, egg; furthermore sphingolipids (e.g. ceramides, cerebrosides, sphingosin, sphingomyelin); as well as mixtures thereof.

Also preferred are active ingredients f) preferably selected from the group comprising ethereal oils, plant extracts, skin care agents, or mixtures thereof; and / or additives e), especially selected amongst dyes, perfumes, colloids, pearlescent agents, solubilising agents or mixtures thereof.

Especially preferred embodiments include agglomerates comprising 87 to 96 % by weight of urea (beads), and:
a1)0.1 to 3 %, in particular 0.1 to 2% by weight of one or more liquid lipids a1), preferably selected from the group comprising olive oil, sunflower oil, soy oil, peach pit oil, apricot pit oil, grape seed oil, castor bean oil, peanut oil, almond oil, mink oil, wheat germ oil, sesame oil, thistle oil, avocado oil, shea butter or illipé butter; natural liquid waxes, e.g. jojoba oil or its substitute, oleyl erucate; coconut oil, borage oil, corn oil, walnut oil, palm oil macademia nut oil, palm germ oil, hazelnut oil, rosehip oil, cotton seed oil, papaya oil, or mixtures thereof; particularly selected amongst soy(bean)oil, sunflower oil, wheat germ oil, olive oil or mixtures thereof; whereby a1) is especially preferably selected from the group of olive oil, sunflower oil, soy (bean) oil, peach pit oil, apricot pit oil, grape seed oil, castor oil, peanut oil, almond oil, or mixtures thereof;
as well as comprising:
a2) 0.4 to 3, especially 0.4 to 2 % by weight, of one or more solid to wax like lipids, particularly selected from hydrogenated soy (bean) oil, hydrogenated palm oil, hydrogenated castor oil, hydrogenated sunflower oil, especially hydrogenated soy (bean) oil;
as well as comprising:
b) 0.1 to 2 % by weight of (one or more of) a vesicle forming lipid(s), especially 0.1 to 1.5 % by weight; of phospholipids, such as lecithin (egg- or soy lecithin); or phosphatidyl-cholin, -serin or -diethanolamine; phosphatidylinosit, e.g. derived from soy, rape, cotton seed, egg;
c) 0.1 to 1.5 % by weight of (one or more of) non ionic dispersion agent(s), selected from ethoxylated C₁₂₋₂₀ -fatty- alcohols having an ethoxylation degree of between 2 and 4; and
d) 0.8 to 2 % by weight of (one or more of) an anionic O/W emulsifier(s), especially an (alkyl) -C₁₂₋₂₂ -fatty- alcohol- amino acid ester compound, especially an alkali metal salt thereof and the amino acid being selected from glycine, taurine;
e) 0.5 to 5% by weight of additives, especially dyes, perfumes, conditioning agents, such as in particular pearlescent agents, wax glossing agents, colloids;
as well as
f) 0.2, to 2 % by weight of active ingredients, especially ethereal oils, skin care agents and / or plant extracts.

Further preferred embodiments include agglomerates comprising 87 to 96 % by weight of urea (beads), and:
a1) 0.1 to 3 %, in particular 0.1 to 2% by weight of one or more liquid lipids as defined in claim 1;
a2) 0.4 to 3, especially 0.4 to 2 % by weight, of one or more solid to wax like lipids a2), particularly selected from hydrogenated soy oil;
b) 0.1 to 2 % by weight of (one or more of) a vesicle forming lipid(s), especially 0.1 to 1.5 % by weight; selected from phosphatidylcholin, lecithin or mixtures thereof;
c) 0.1 to 1.5 % by weight of (one or more of) an ethoxylated C₁₂₋₂₀ -fatty- alcohol having an ethoxylation degree of between 1 and , especially between of 2 and 4,
d) 0.8 to 2 % by weight of (one or more of) an anionic O/W emulsifier(s), selected from (alkyl) -C₁₂₋₂₂ -fatty- alcohol- amino acid ester compounds, especially an alkali metal salt thereof, and the amino acid being selected from glycine, taurine;
e) 0.5 to 5% by weight of (one or more) additives, especially dyes, perfumes, conditioning agents, such as in particular pearlescent agents, colloids;
   as well as
f) 0.2, to 2 % by weight of active ingredients, especially ethereal oils, skin-care agents and / or plant extracts.

A specifically preferred embodiment includes agglomerates comprising 83 to 96 % by weight of urea (or urea beads/prills);
a1) 0.1 to 2% by weight of one or more liquid lipids a1) such as almond oil, jojoba oil;
a2) 0.4 to 1.5 % by weight, of a solid lipid,a2)
b) 0.1 to 2 % by weight of soy or egg lecithin, phosphatidyl-cholin or -serin, or mixtures thereof; in particular soy lecithin;
c) 0.1 to 1 % by weight of an ethoxylated C₁₂₋₁₈ -fatty- alcohol having an ethoxylation degree of between 2 and 4 EO;
d) 0.8 to 1,5 % by weight of an anionic O/W emulsifier (anionic tenside), especially an (alkyl) -C₁₂₋₂₂ -fatty- alcohol- amino acid ester compound, especially an alkali metal salt thereof;
e) 0.5 to 3% by weight of (one or more) additives, especially dyes, perfumes, conditioning agents, in particular colloids such as silica acid derivatives, dextrins, pearlescent agents, wax glossing agents; dyes, perfumes;
   as well as
f) 0.2 to 2 % by weight of active ingredients, especially ethereal oils, skin-care agents and / or plant extracts.

The agglomerates according to the invention preferably have an average diameter of about 0.5 to 5 mm, in particular of 1 to 3 mm, and are preferably present in the form of bath beads.

### Production and use

The agglomerates according to the invention are prepared by treating a preferably spherically formed, essentially anhydrous carrier, especially urea, in a mixing vessel 1) with a part of a (separately prepared in a known manner) liquid to fluid mixture 1) of the liquid (if present) lipid a1), the vesicle forming lipid b), the dispersion agent c) and optional perfumes e), optional liquid compound d) and optional active ingredients e), preferably at room temperature (e. g. by applying, for example such as spraying, trickling);
2) contacting a mixture of the solid component(s) a2), O/W emulsifier d), optional solid active ingredients, optional solid additives such as colloids, in the mixing vessel with the carrier comprising the liquid mixture as obtained by step 1 and mixing the same; and then
3) applying the balance of the liquid to fluid mixture 1) onto the carrier obtained in step 2) and optionally treating the same with additives as far as present, selected from conditioning agents, especially colloids, pearlescent agents, wax glossing agents, dyes, solubilising agents, or mixtures thereof, in the mixing vessel (e.g. by application for example such as trickling, spraying).

Preferably, in step 1) also a liquid lipid a1) and in step 2) a lipid a2) is used.

Insofar component d) is liquid to fluid, or a mixture of liquid and solid substances, the liquid part is incorporated into the mixture 1.

It is preferred to apply about 50 to 80% by weight, especially 60-70% by weight, of the mixture 1 in step 1 and the balance in step 3).

By such a proceeding stable agglomerates are obtained, in particular bath beads having an essentially spherical form, which preferably do not comprise any preservation agents and which are essentially anhydrous.

Such agglomerates may easily be packed into suitable containers and transported in a most comfortable manner without loosing the agglomeration properties. As a consequence they may be easily applied to the bathing by adding the desired amount.

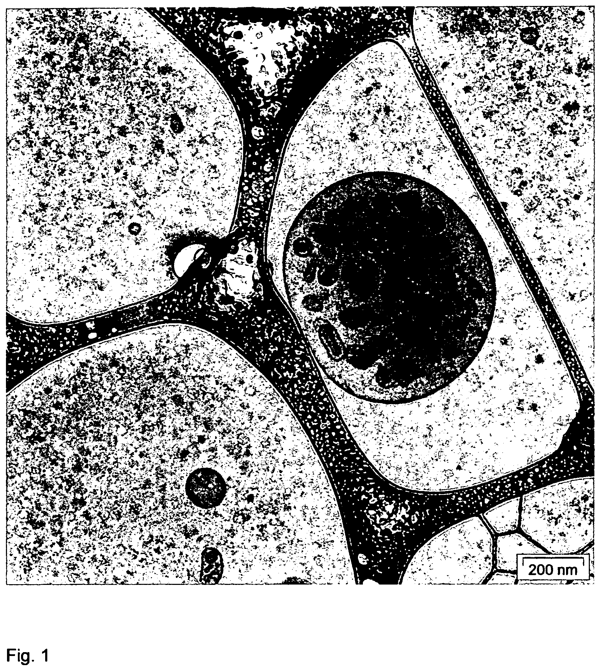

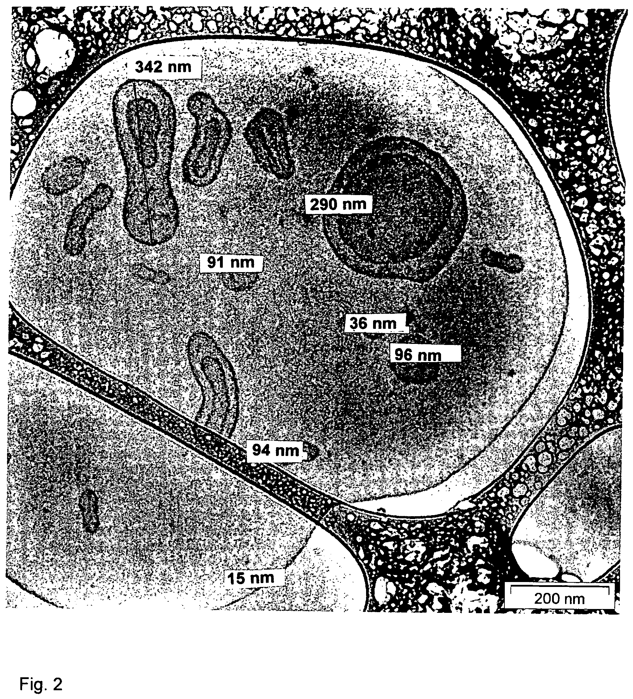

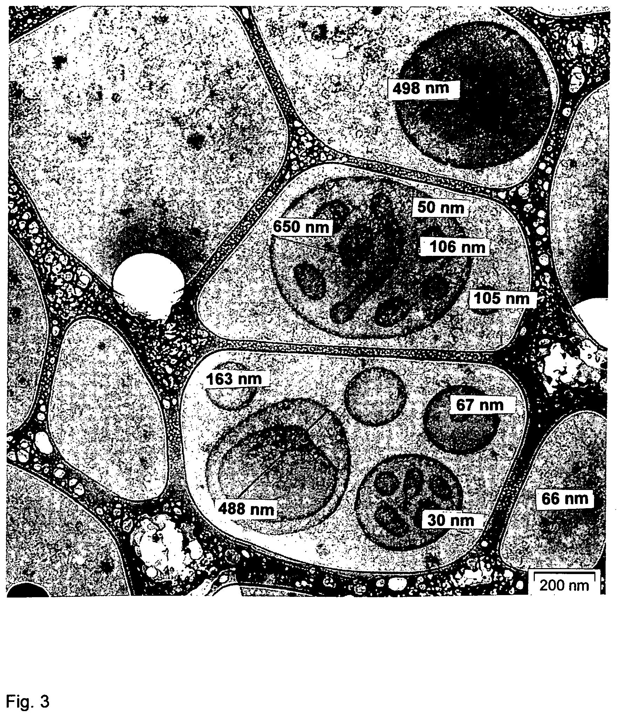

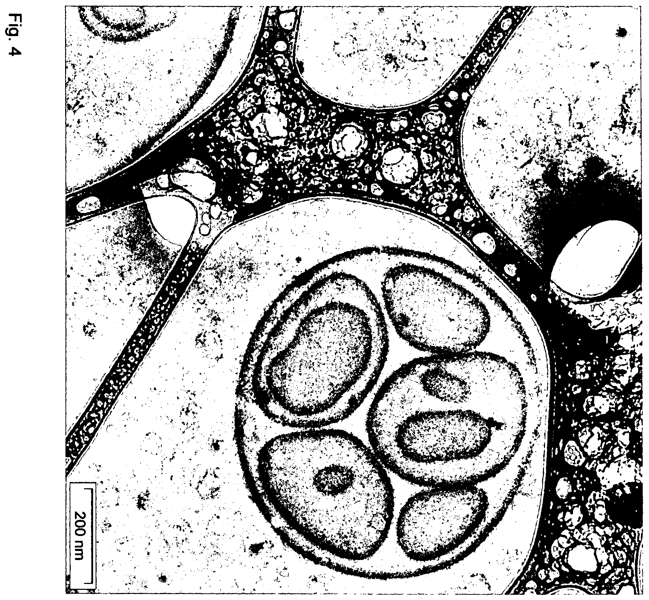

If added to a bathing area the agglomerates are rapidly solved, whereby surprisingly specific liposomal mono - to multilayer-structures are formed. These are shown in the electron photomicro-graphs (TEM photographs) according to Fig. 1 to 4. The formation of such structures allows a high caring effect, since on the one hand active ingredients such as lipidic agents, vitamins and the like, may be transferred more easily due to the liposomal mono- to multilayer-structures. On the other hand there is an increase through the natural carrier, which itself has a cutaneous, in particular, caring / refatting and therefore reconstructive effect on the corneal skin layer.

The agglomerates according to the invention consequently are usable as additives to full, part, aroma, refreshing or feet bath.

They may be applied in a cosmetic bath e.g. as a relaxing, or as a hygienic bathing or even therapeutic bathing. Insofar a sufficient amount of the agglomerates, preferably the beads, specifically the urea beads, which may comprise suitable active ingredients in view of the desired purpose, e.g. ethereal oil for refreshing, relaxing, ingredients for blood circulation, anti- bacterial / anti- inflammatory agents as described hereinabove, are applied to the desired bathing having a temperature suitable for the desired purpose, for example 35- 38° C, or otherwise adapted (feet bathing).

The invention is further illustrated (without limitation) by the following examples 1-5.

Thereby the agglomerates described have been prepared as indicated above.

### Example 1

| Compound | Wt.% |
|---|---|
| microcristalline cellulose pellets | 93 |
| Palm oil, hydrogenated | 2 |
| Almond oil | 1 |
| Phosphatidylcholin | 1 |
| Cetearyl alcohol | 1 |
| Natrium methyl oleyl taurate | 1 |
| Perfume oil | 0,5 |
| Dye | 0,1 |
| Cera Alba | 0,2 |
| Fig fruit powder extract | 0,2 |
| | 100 |

### Example 2

| Compound | Wt.% |
|---|---|
| mikrocristalline rice starch pellets | 86,9 |
| Coconut oil, hydrogenated | 4 |
| Grape germ oil | 1 |
| Soy lecithin | 2 |
| Glyceryl stearate | 1,5 |
| Polyoxyethylenesorbitan-monooleate (HLB=15; EO≈20) | 2 |
| Perfume oil | 1,5 |
| Dye | 0,1 |
| Dextrine | 0,5 |
| Jasmine flower extract | 0,5 |
| | 100 |

### Example 3

| Compound | Wt.% |
|---|---|
| Urea beads (prills) | 89,7 |
| Macademia nut oil, hydrogenated | 2,4 |
| Plum germ oil | 0,8 |
| Polyoxyethylenesorbitan-monolaurate (HLB=16,7; EO≈20) | 1 |
| Lecithin, derived from cotton seed | 1,5 |
| Laureth-3 (HLB=8, EO=3) | 0,5 |
| Perfume oil | 1,5 |
| Dye | 0,1 |
| Methyl cellulose | 0,5 |
| Vanilla extrakt | 0,8 |
| Pine needle oil | 1,2 |
| | 100 |

### Example 4

| Compound | Wt.% |
|---|---|
| Collagen hydrolyzate pellets | 89,2 |
| Castor oil, hydrogenated | 2 |
| Wheat germ oil | 0,5 |
| Polyoxyethylene sorbitan monooleate (HLB=15, EO≈20) | 2 |
| Phosphatidylserine | 1,5 |
| Laureth-4 (HLB=9,5; EO=4) | 2 |
| Perfume oil | 1,2 |
| Dye | 0,1 |
| Silicium dioxide | 0,5 |
| Ivy leaf extract | 1 |
| | 100 |

### Example 5

| Compound | Wt.% |
|---|---|
| Urea beads | 88,7 |
| Sunflower oil, hydrogenated | 3,2 |
| Shea Butter | 0,6 |
| Polyoxyethylene laurylether (HLB=14,5; EO≈12) | 2 |
| Rapeseed Lecithin | 2 |
| Laureth-2 (HLB=7; EO=2) | 1 |
| Perfume | 0,6 |
| Dye | 0,1 |
| Hydroxypropyl cellulose | 0,4 |
| Salicylic acid | 0,8 |
| Winter green oil | 0,6 |
| | 100 |

## Claims

1. Isometric balneological agglomerates having a nearly spherical form, comprising:
80 to 97 % by weight of a carrier, selected from the group of collagen hydrolyzate originating from plants, animals or marine sources; gelatine or gelatine hydrolyzate; cellulose; carbohydrates, starch or urea; as well as
a) 0.1 % by weight up to 10 % by weight - in relation to the total amount of lipids - of a mixture of:
a1) one or more liquid lipids, selected from the group of olive oil, sunflower oil, soy (bean) oil, peach pit oil, apricot pit oil, grape seed oil, castor oil, peanut oil, almond oil, mink oil, wheat germ oil, sesame oil, thistle oil, avocado oil, shea butter or illipé butter; natural liquid waxes, e.g. jojoba oil or its substitute, oleyl erucate; coconut oil, borage oil, corn oil, walnut oil, palm oil, hazelnut oil, rosehip oil, cotton seed oil, papaya (paw paw) oil, or
mixtures thereof, and
a2) one or more solid up to waxy like lipids;
b) 0.1 up to 2 % by weight of one or more vesicle forming lipids, selected from phospholipids such as lecithin (e.g. egg or soy lecithin), or phosphatidyl-choline, -serine, or -diethanolamine, phosphatidylinosite, e g. derived from soy, rape, cotton seed, egg; sphingolipids (e.g., sphingosine, ceramides, cerebrosides, sphingomyelin), or ethoxylated sterols, or mixtures thereof;
c) 0.1 up to 5% by weight of a one or more dispersion agent (s) having an HLB value of between 7 and 11;
d) 0,5 up to 6 % by weight of one or more emulsifier compound(s) having an HLB value of especially between 13 and 40, more specifically between 13-25.

2. Agglomerates according to claims 1 **characterized in that** they additionally comprise e) 0 up to 10 %, preferably 0.1 up to 10 % by weight of additives selected from the group comprising perfumes, dyes, conditioners, and / or f) 0 up to 5 %, preferably 0.1 up to 5% by weight of active ingredients.

3. Agglomerates according to claim 1 or 2 **characterized in that** the carrier is selected from the group of urea, in particular spherically formed urea beads.

4. Agglomerates according to any of claims 1 to 3 **characterized in that** the solid lipid(s) a2) is (are) selected from the group of hydrogenated soy (bean) oil, hydrogenated palm oil, hydrogenated castor oil, hydrogenated sunflower oil, or mixtures thereof.

5. Agglomerates according to any of claims 1 to 4 **characterized in that** the dispersion agent(s) c) is (are) selected from the group of non ionic low polyethoxylated or low polypropoxylated and low - mixed polyoxylated, C₈₋₂₂ -fatty- alcohols having an ethoxylation (and / or propoxylation) degree (EO/PO degree) of between 1 and 5; ethoxylated glyceroles, being esteryfied with C₁₂₋₂₂-fatty- acids and having an EO degree of between 0.5 and 3.

6. Agglomerates according to any of claims 1 to 5 **characterized in that** the O/W emulsifier(s) d) is (are) selected from the group of higher ethoxylated C₁₂₋₂₂ -fatty- acids or higher ethoxylated C₈₋₂₂ -fatty- alcohols having ethoxylation degrees of between 13 and 18 and an HLB- value of between 13.5 and 15; sugar esters or sugar ethers of C₁₂₋₂₂ -fatty- acids or of C₈₋₂₂ -fatty-alcohols; polyoxyethylated and / or polyoxypoxylated sugar esters of C₁₂₋₁₈ - fattyacids having an EO and PO degree, respectively of between 8 and 25 and the sugar being derived from glucose, saccharose, methyl glucose; as well as alkyl C₁₂₋₂₂ -fatty- alcohol- amino acid ester- compounds, especially an alkali metal salt thereof, the amino acid being selected amongst glycine and taurine.

7. Agglomerates according to any of claims 1 to 6 **characterized in that** the ratio between the O/W emulsifier(s) d) and the dispersion agent(s) c) and the is > 1.

8. Agglomerates according to any of claims 1 to 7 **characterized in that** the amount of the solid to wax like lipid(s) a2) is higher than the amount of the liquid lipid(s) a1).

9. Agglomerates according to any of claims 1 to 8 **characterized in that** they comprise active ingredients f), selected from ethereal oils, plant extracts, skin care agents, or mixtures thereof.

10. Agglomerates according to any of claims 1 to 9 **characterized in that** they comprises additives e) selected from dyes, perfumes, conditioning agents, selected from colloids, pearlescent agents, solubilising agents; or mixtures thereof.

11. Agglomerates according to any of claims 1 to 10 **characterized in that** they comprise 87 to 96 % by weight of urea (beads), and:
a1) 0.1 to 3 % by weight of one or more liquid lipids a1);
a2) 0.4 to 3 % by weight, of one or more solid to wax like lipids, particularly selected from hydrogenated soy (bean) oil;
b) 0.1 to 2 % by weight of (one or more) a vesicle forming lipid, selected from phosphatidylcholin, lecithin or mixtures thereof;
c) 0.1 to 1.5 % by weight of an ethoxylated C₁₂₋₂₀ -fatty- alcohol having an ethoxylation degree of between 2 and 4 EO;
d) 0.8 to 2 % by weight of an anionic O/W emulsifier, selected from (alkyl) - (C₁₂₋₂₂ -fatty- alcohol- amino acid ester compounds, especially an alkali metal salt thereof, and the amino acid being selected from glycine, taurine;
e) 0,5 to 5% by weight of (one or more) additives, especially dyes, perfumes, conditioning agents, such as in particular pearlescent agents, wax glossing agents, colloids;
as well as
f) 0.2, to 2 % by weight of active ingredients, especially ethereal oils, skin-care agents and / or plant extracts.

12. The agglomerates according to any of claims 1 to 11 **characterized in that** they have an average diameter of about 0.5 to 5 mm.

13. Process for producing the agglomerates according to any of claims 1 to 12 **characterized in** by treating a spherically formed, essentially anhydrous carrier, especially urea, in a mixing vessel
1) with a part of a (separately prepared in an known manner) liquid to fluid mixture 1) of the liquid lipid(s) a1), vesicle forming lipid(s) b), dispersing agent(s) c) and optional perfumes e) optional liquid compound(s) d) and optional active ingredients f), preferably at room temperature;
2) contacting a mixture of the solid components a2), O/W emulsifier(s) d), optional active ingredients, optional solid additives such as colloids, in the mixing vessel with the carrier comprising the mixture 1) as obtained by step 1 and mixing the same; and then
3) applying the balance of the liquid to fluid mixture 1) onto the carrier obtained in step 2) and optionally treating the same with additives as far as present, selected from conditioning agents, especially colloids, pearlescent agents, wax glossing agents, dyes, solubilizing agents, or mixtures thereof, in the mixing vessel.

14. Use of agglomerates according to any of claims 1 to 12 or prepared according to claim 13 as additives for cosmetic or hygienic aroma, refreshing or feet bathings.

15. Use of agglomerates according to any of claims 1 to 12 for preparing agents as additives for therapeutic or hygienic aroma, refreshing or feet bathings.

## Patentansprüche

1. Isometrische balneologische Agglomerate mit annährend kugeliger Form, umfassend:
80 bis 97 Gew.% eines Trägers, ausgewählt aus der Gruppe aus Kollagenhydrolysat aus pflanzlichen, tierischen oder marinen Quellen; Gelatine oder Gelatinehydrolysat; Cellulose; Kohlenhydraten, Stärke oder Harnstoff; sowie
a) 0,1 Gew.% bis 10 Gew.% - bezogen auf die Gesamtlipidmenge - einer Mischung aus:
a1) einem oder mehreren flüssigen Lipiden, ausgewählt aus der Gruppe aus Olivenöl, Sonnenblumenöl, Soja(bohnen)öl, Pfirsichkernöl, Aprikosenkernöl, Traubenkernöl, Ricinusöl, Erdnussöl, Mandelöl, Nerzöl, Weizenkeimöl, Sesamöl, Distelöl, Avocadoöl, Shea Butter oder Illipé Butter; natürlichen flüssigen Wachsen, z. B. Jojobaöl oder dessen Substitut, Oleyl Erucate; Kokosnussöl, Borretschöl, Maiskeimöl, Walnussöl, Palmöl, Haselnussöl, Hagebuttenöl, Baumwollsamenöl, Papayaöl, (Papaya) oder Mischungen hiervon,
und
a2) einem oder mehreren festen bis wachsartigen Lipiden;
b) 0,1 bis 2 Gew.% eines oder mehrerer vesikelbildender Lipide, ausgewählt aus Phospholipiden, wie Lecithin (z. B. Ei- oder Soja-Lecithin), oder Phosphatidylcholin, -serin oder -diethanolamin, Phospatidylinosit, z. B. aus Soja, Raps, Baumwollesamen, Ei; Sphingolipiden (z. B. Sphingosin, Ceramide, Cerebroside, Sphingomyelin) oder ethoxylierten Sterolen, oder Mischungen hiervon;
c) 0,1 bis 5 Gew.% eines oder mehrerer Dispersionsmittel(s) mit einem HLB-Wert von zwischen 7 und 11;
d) 0,5 bis 6 Gew.% einer (oder mehrerer) Emulgatorkomponente(n) mit einem HLB-Wert von vor allem zwischen 13 und 40, insbesondere zwischen 13 - 25.

2. Agglomerate gemäß Anspruch 1, **dadurch gekennzeichnet, dass** sie weiterhin e) 0 bis 10, vor allem 0,1 bis 10 Gew.-% Zusatzstoffe, ausgewählt aus der Gruppe, umfassend Parfümstoffe, Farbstoffe, Konditioniermittel, und/oder f) 0 bis 5 %, vorzugsweise 0,1 bis 5 Gew.% Wirkstoffe aufweisen.

3. Agglomerate gemäß Anspruch 1 oder 2, **dadurch gekennzeichnet, dass** der Träger ausgewählt ist aus der Gruppe aus Harnstoff, insbesondere sphärisch geformten Harnstoffperlen.

4. Agglomerate gemäß einem der Ansprüche 1 bis 3, **dadurch gekennzeichnet, dass** das/ die festen Lipid(e) a2) ausgewählt ist (sind) aus der Gruppe aus hydriertem Soja(bohnen)öl, hydriertem Palmöl, hydriertem Rizinusöl, hydriertem Sonnenblumenöl oder Mischungen hiervon.

5. Agglomerate gemäß einem der Ansprüche 1 bis 4, **dadurch gekennzeichnet dass**, das /die Dispersionsmittel c) ausgewählt ist (sind) aus der Gruppe aus nichtionischen niedrigpolyethoxylierten oder niedrig polypropoxylierten und niedrig gemischtpolyoxylierten C₈₋₂₂-Fettalkoholen mit einem Grad an Ethoxylierung (und/oder Propoxylierung) (EO/PO-Grad) von zwischen 1 und 5, ethoxylierten Glycerolen, welche mit C₁₂₋₂₂-Fettsäuren verestert sind und einen EO-Grad von zwischen 0,5 und 3 aufweisen.

6. Agglomerate gemäß einem der Ansprüche 1 bis 5, **dadurch gekennzeichnet, dass** der/die O/W Emulgator(en) d) ausgewählt ist (sind) aus der Gruppe aus höher ethoxylierten C₁₂₋₂₂-Fettsäuren oder höher ethoxylierten C₈₋₂₂-Fettalkoholen mit Ethoxylierungsgraden von zwischen 13 und 18 und einem HLB-Wert von zwischen 13,5 und 15; Zuckerestern oder Zuckerethern von C₁₂₋₂₂ -Fettsäuren oder von C₈₋₂₂-Fettalkoholen; polyoxyethylierten und/ oder polyoxypropylierten Zuckerestern von C₁₂₋₁₈-Fettsäuren mit einem EO bzw. PO Grad von zwischen 8 und 25 und wobei der Zucker abgeleitet ist von Glucose, Saccharose, Methyl-glukose; sowie (Alkyl)- C₁₂₋₂₂ -Fettalkohol-Aminosäureester-Verbindungen, insbesondere einem Alkalimetallsalz hiervon, wobei die Aminosäure ausgewählt ist aus Glycin und Taurin.

7. Agglomerate gemäß einem der Ansprüche 1 bis 6, **dadurch gekennzeichnet, dass** das Verhältnis zwischen O/W-Emulgator(en) d) und Dispersionsmittel(n) c) > 1 ist.

8. Agglomerate gemäß einem der Ansprüche 1 bis 7, **dadurch gekennzeichnet, dass** die Menge an festen bis wachsartigen Lipid(en) a2) höher ist als die Menge an flüssigem(n) Lipid(en) a1).

9. Agglomerate gemäß einem der Ansprüche 1 bis 8, **dadurch gekennzeichnet, dass** sie Wirkstoffe f) umfassen, ausgewählt aus ätherischen Ölen, Pflanzenextrakten, Hautpflegestoffen, oder Mischungen hiervon.

10. Agglomerate gemäß einem der Ansprüche 1 bis 9, **dadurch gekennzeichnet, dass** sie Zusatzstoffe e) umfassen, ausgewählt aus Farbstoffen, Parfümstoffen, Konditioniermitteln, ausgewählt aus Kolloiden, Perlglanzmitteln, Lösungsvermittlern; oder Mischungen hiervon.

11. Agglomerate gemäß einem der Ansprüche 1 bis 10, **dadurch gekennzeichnet, dass** sie 87 bis 96 Gew.% Harnstoff (Perlen) umfassen und:
a1) 0,1 bis 3 Gew.% eines oder mehrerer flüssiger Lipide a1),
a2) 0,4 bis 3 Gew.% eines oder mehrerer fester bis wachsartiger Lipide, vor allem ausgewählt aus hydriertem Soja(bohnen)öl;
b) 0,1 bis 2 Gew.% eines (oder mehrerer) vesikelbildenden Lipids, ausgewählt aus Phosphatidylcholin, Lecithin oder Mischungen hiervon;
c) 0,1 bis 1,5 Gew.% eines ethoxylierten C₁₂₋₂₀ -Fettalkoholes mit einem Ethoxylierungsgrad von zwischen 2 und 4 EO;
d) 0,8 bis 2 Gew.% eines anionischen O/W Emulgators, ausgewählt aus (Alkyl)- C₁₂₋₂₂-Fettalkohol-Aminosäureester-Verbindungen, insbesondere einem Alkalimetallsalz hiervon, wobei die Aminosäure ausgewählt ist aus Glycin, Taurin;
e) 0,5 bis 5 Gew.% (eines oder mehrerer) Zusatzstoffe, vor allem Farbstoffe, Parfümstoffe, Konditioniermittel, wie vor allem Perlglanzmittel, Wachsglanzmittel, Kolloide;
sowie
f) 0,2 bis 2 Gew.% Wirkstoffe, vor allem ätherische Öle, Hautpflegestoffe und/oder Pflanzenextrakte.

12. Agglomerate gemäß einem der Ansprüche 1 bis 11, **dadurch gekennzeichnet, dass** sie einen mittleren Durchmesser von 0,5 bis 5 mm aufweisen.

13. Verfahren zur Herstellung von Agglomeraten gemäß einem der Ansprüche 1 bis 12, **dadurch gekennzeichnet, dass** man einen sphärisch geformten, im wesentlichen wasserfreien Träger, vor allem Harnstoff, in einem Mischer
1) mit einem Teil einer (in an sich bekannter Weise separat hergestellten) flüssigen bis fließfähigen Mischung 1) aus flüssigem Lipid(en) a1), vesikelbildendem Lipid(en) b), Dispersionsmittel(n) c) und wahlweise Parfümstoffen e), wahlweise flüssigen Komponente(n) d) und wahlweise Wirkstoffen f), vorzugsweise bei Raumtemperatur behandelt;
2) eine Mischung aus den festen Bestandteilen a2), dem(n) O/W Emulgator(en) d), wahlweise Wirkstoffen, wahlweise festen Zusatzstoffen wie Kolloiden, im Mischer mit dem Träger, umfassend die Mischung 1) wie in Schritt 1 erhalten, in Kontakt bringt und mischt; und anschließend
3) den Rest der flüssigen bis fließfähigen Mischung 1) auf den in Schritt 2) erhaltenen Träger aufbringt und diesen wahlweise mit Zusatzstoffen, sofern vorhanden, ausgewählt aus Konditioniermitteln, vor allem Kolloiden, Perlglanzmitteln, Wachsglanzmitteln, Farbstoffen, Lösungsvermittlern, oder Mischungen hiervon, im Mischer behandelt.

14. Verwendung von Agglomeraten gemäß einem der Ansprüche 1 bis 12 oder hergestellt nach Anspruch 13 als Zusätze für kosmetische oder hygienische Aroma, Erfrischungs- oder Fußbäder.

15. Verwendung von Agglomeraten gemäß einem der Ansprüche 1 bis 12 zur Herstellung von Mitteln als Zusätze für therapeutische oder hygienische Aroma, Erfrischungs- oder Fußbäder.

## Revendications

1. Agglomérats balnéologiques isomères présentant une forme presque sphérique, comprenant :
de 80 à 97 % en poids d'un support, sélectionné dans le groupe de l'hydrolysat de collagène provenant de végétaux, d'animaux ou de sources marines ; de la gélatine ou de l'hydrolysat de gélatine ; de la cellulose ; des hydrates de carbone, de l'amidon ou de l'urée ; ainsi que
a) de 0,1 % en poids jusqu'à 10 % en poids - en lien avec la quantité totale de lipides - d'un mélange de :
a1) un ou plusieurs lipides liquides, sélectionnés dans le groupe de l'huile d'olive, de l'huile de tournesol, de l'huile de soja (haricot), de l'huile de noyau de pêche, de l'huile de noyau d'abricot, de l'huile de pépins de raisin, de l'huile de ricin, de l'huile d'arachide, de l'huile d'amande, de l'huile de vison, de l'huile de germe de blé, de l'huile de sésame, de l'huile de chardon, de l'huile d'avocat, de l'huile de karité ou de l'huile de suif de Bornéo ; des cires liquides d'origine naturelle, par exemple de l'huile de jojoba ou son substitut, d'érucate d'oléyle ; de l'huile de noix de coco, de l'huile de bourrache, de l'huile de maïs, de l'huile de noix, de l'huile de palme, de l'huile de noisette, de l'huile de bouton de rose, de l'huile de graines de coton, de l'huile de papaye (papayer), ou
de leurs mélanges, et
a2) d'un ou plusieurs lipides solides jusqu'aux lipides semblables à de la cire ;
b) de 0,1 jusqu'à 2 % en poids d'un ou plusieurs lipides formant des vésicules, sélectionnés parmi les phospholipides tels que la lécithine (par exemple la lécithine de jaune d'oeuf ou de soja), ou la phosphatidylcholine,-sérine, ou -diéthanolamine, phosphatidylinosite, par exemple dérivés du soja, du colza, des graines de coton, d'oeuf ; des sphingolipides (par exemple, sphingosine, céramides, cérébrosides, sphingomyéline), ou des stérols éthoxylés, ou de leurs mélanges;
c) de 0,1 jusqu'à 5% en poids d'un ou plusieurs agent(s) de dispersion présentant un indice d'équilibre lipophile-hydrophile, HLB compris entre 7 et 11 ;
d) de 0,5 jusqu'à 6 % en poids d'un ou plusieurs composé(s) émulsifiant(s) ayant un indice d'équilibre lipophile-hydrophile particulièrement compris entre 13 et 40, plus spécifiquement entre 13 à 25.

2. Agglomérats selon la revendication 1 **caractérisés en ce qu'**ils comprennent additionnellement e) de 0 jusqu'à 10 %, préférablement de 0,1 jusqu'à 10 % en poids d'additifs sélectionnés dans le groupe comprenant les parfums, les colorants, les agents de conditionnement, et/ou f) de 0 jusqu'à 5 %, préférablement de 0,1 jusqu'à 5% en poids d'ingrédients actifs.

3. Agglomérats selon la revendication 1 ou 2 **caractérisés en ce que** le support est sélectionné dans le groupe de l'urée, en particulier des billes d'urée sphériquement formées.

4. Agglomérats selon l'une quelconque des revendications 1 à 3 **caractérisés en ce que** le(s) lipide(s) solide(s) a2) est(sont) sélectionné(s) dans le groupe de l'huile de soja (haricot) hydrogénée, de l'huile de palme hydrogénée, de l'huile de ricin hydrogénée, de l'huile de tournesol hydrogénée, ou de leurs mélanges.

5. Agglomérats selon l'une quelconque des revendications 1 à 4 **caractérisés en ce que** l'(les) agent(s) de dispersion c) est(sont) sélectionné(s) dans le groupe des alcools gras en C₈ à C₂₂ non ioniques inférieurs polyéthoxylés ou inférieurs polypropoxylés et inférieurs-polyoxylés mixtes, ayant un degré d'éthoxylation (et/ou de propoxylation) (degré d'EO/PO) compris entre 1 et 5 ; des glycérols éthoxylés, étant estérifiés avec des acides gras en C₁₂ à C₂₂ et présentant un degré d'EO compris entre 0,5 et 3.

6. Agglomérats selon l'une quelconque des revendications 1 à 5 **caractérisés en ce que** l'(les) agent(s) émulsifiant(s) huile-dans-eau d) est(sont) sélectionné(s) dans le groupe des acides gras en C₁₂ à C₂₂ supérieurs éthoxylés ou des alcools gras en C₈ à C₂₂ supérieurs éthoxylés présentant des degrés d'éthoxylation compris entre 13 et 18 et un indice d'équilibre lipophile-hydrophile compris entre 13,5 et 15 ; des esters de sucre ou des éthers de sucre d'acides gras en C₁₂ à C₂₂ ou d'alcools gras en C₈ à C₂₂ ; des esters de sucre polyoxyéthylés et/ou polyoxypropoxylés d'acides gras en C₁₂ à C₁₈ - présentant un degré d'EO et PO, respectivement compris entre 8 et 25 et le sucre étant dérivé du glucose, du saccharose, du méthyl glucose ; ainsi que des composés ester d'acide aminé, d'alcool gras, d'alkyle en C₁₂ à C₂₂, particulièrement leur sel de métal alcalin, l'acide aminé étant sélectionné parmi la glycine et la taurine.

7. Agglomérats selon l'une quelconque des revendications 1 à 6 **caractérisés en ce que** le rapport entre l'(les) agent(s) émulsifiant(s) huile-dans-eau d) et l'(les) agent(s) de dispersion c) est supérieur à > 1.

8. Agglomérats selon l'une quelconque des revendications 1 à 7 **caractérisés en ce que** la quantité de lipide(s) solide(s) jusqu'aux lipides semblables à de la cire a2) est supérieure à la quantité de lipide(s) liquide(s) a1).

9. Agglomérats selon l'une quelconque des revendications 1 à 8 **caractérisés en ce qu'**ils comprennent les ingrédients actifs f), sélectionnés parmi les huiles éthérées, les extraits végétaux, les agents de soin de la peau, ou leurs mélanges.

10. Agglomérats selon l'une quelconque des revendications 1 à 9 **caractérisés en ce qu'**ils comprennent les additifs e) sélectionnés parmi les teintures, les parfums, les agents de conditionnement, sélectionnés parmi les colloïdes, les agents perlescents, les agents de solubilisation ; ou leurs mélanges.

11. Agglomérats selon l'une quelconque des revendications 1 à 10 **caractérisés en ce qu'**ils comprennent de 87 à 96 % en poids d'urée (billes), et :
a1) de 0,1 à 3 % en poids d'un ou plusieurs lipides liquides a1) ;
a2) de 0,4 à 3 % en poids, d'un ou plusieurs lipides solides jusqu'aux lipides semblables à de la cire particulièrement sélectionnés parmi l'huile de soja (haricot) hydrogénée ;
b) de 0,1 à 2 % en poids d'(un ou plusieurs) lipides formant des vésicules, sélectionnés parmi la phosphatidylcholine, la lécithine ou leurs mélanges ;
c) de 0,1 à 1,5 % en poids d'un alcool gras en C₁₂ à C₂₀ éthoxylé présentant un degré d'éthoxylation compris entre 2 et 4 EO ;
d) de 0,8 à 2 % en poids d'un agent émulsifiant huile-dans-eau anionique, sélectionné parmi les composés ester d'acide aminé d'(alcool gras en C₁₂ à C₂₂)-(alkyle), particulièrement son sel de métal alcalin, et l'acide aminé étant sélectionné parmi la glycine, la taurine ;
e) de 0,5 à 5 % en poids d'(un ou plusieurs) additifs, particulièrement des teintures, des parfums, des agents de conditionnement, tels qu'en particulier des agents perlescents, des agents de brillance cireux, des colloïdes ;
ainsi que
f) de 0,2, à 2 % en poids d'ingrédients actifs, particulièrement des huiles éthérées, des agents de soin de la peau et/ou des extraits végétaux.

12. Agglomérats selon l'une quelconque des revendications 1 à 11 **caractérisés en ce qu'**ils présentent un diamètre moyen d'environ 0,5 à 5 mm.

13. Procédé de production des agglomérats selon l'une quelconque des revendications 1 à 12 **caractérisés par** le traitement d'un support essentiellement anhydre, sphériquement formé, spécialement de l'urée, dans un récipient de mélange
1) avec une partie d'un liquide (préparé séparément d'une manière connue) en un mélange fluide 1) du(des) lipide(s) liquide(s) a1), de lipide(s) formant des vésicules b), d'agent(s) de dispersion c) et de parfums facultatifs e) de composé(s) liquide(s) facultatif(s) d) et d'ingrédients actifs facultatifs f), préférablement à la température ambiante ;
2) la mise en contact d'un mélange des constituants solides a2), d'émulsifiant(s) huile-dans-eau d), d'ingrédients actifs facultatifs, d'additifs solides facultatifs tels que des colloïdes, dans le récipient de mélange avec le support comprenant le mélange 1) tel qu'obtenu par l'étape 1 et son mélange ; et ensuite
3) l'application du reste du liquide au mélange fluide 1) sur le support obtenu dans l'étape 2) et son traitement facultativement avec des additifs tant qu'ils sont présents, sélectionnés parmi les agents de conditionnement, particulièrement les colloïdes, les agents perlescents, les agents de brillance cireux, les teintures, les agents de solubilisation, ou leurs mélanges, dans le récipient de mélange.

14. Utilisation des agglomérats selon l'une quelconque des revendications 1 à 12 ou préparés selon la revendication 13 comme additifs pour bains cosmétique ou d'hygiène arôme, rafraîchissants ou de pied.

15. Utilisation des agglomérats selon l'une quelconque des revendications 1 à 12 pour la préparation d'agents comme additifs pour bains thérapeutique ou d'hygiène arôme, rafraîchissants ou de pied.
